(19) Europäisches Patentamt
European Patent Office
Office européen des brevets

(11) Veröffentlichungsnummer: 0 330 962
A2

(12) EUROPÄISCHE PATENTANMELDUNG

(21) Anmeldenummer: 89102945.6

(22) Anmeldetag: 21.02.89

(51) Int. Cl.[4]: C07C 45/46 , C07C 49/813 , C07C 49/84 , C07C 147/06 , C07C 149/34

(30) Priorität: 02.03.88 DE 3806656

(43) Veröffentlichungstag der Anmeldung: 06.09.89 Patentblatt 89/36

(84) Benannte Vertragsstaaten: BE CH DE FR GB IT LI NL

(71) Anmelder: HOECHST AKTIENGESELLSCHAFT
Postfach 80 03 20
D-6230 Frankfurt am Main 80(DE)

(72) Erfinder: Fuss, Andreas, Dr.
Lindigstrasse 24
D-8757 Karlstein(DE)
Erfinder: Siegemund, Günter, Dr.
Frankfurter Strasse 21
D-6238 Hofheim am Taunus(DE)

# (54) Verfahren zur Herstellung von halogenhaltigen aromatischen Verbindungen.

(57) Verfahren zur Herstellung von halogenhaltigen aromatischen Verbindungen der Formel

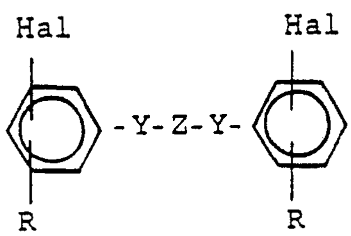

(I),

worin bedeuten
Hal Halogen,
R H, $C_1$-$C_3$-Alkyl, Phenyl, Halogen oder Trifluormethyl,
Y CO oder $SO_2$ und
Z eine aromatische oder heteroaromatische Gruppierung, wobei Y-Z-Y- zusammen auch CO oder $SO_2$ sein können,
dadurch gekennzeichnet, daß man einen Halogenaromaten der Formel $RC_6H_4Hal$ (II) mit einem Bissäurehalogenid der Formel Hal-Y-Z-Y-Hal (III) im Molverhältnis von mindestens 2 : 1 in Gegenwart von Fluorwasserstoff und von Bortrifluorid umsetzt, wobei in den Formeln II und III Hal, R, Y und Z die zuvor angegebene Bedeutung haben.

EP 0 330 962 A2

## Verfahren zur Herstellung von halogenhaltigen aromatischen Verbindungen

Die Erfindung bezieht sich auf ein Verfahren zur Herstellung von halogenhaltigen aromatischen Verbindungen, die mindestens zwei aromatische Ringe enthalten und über wenigstens ein Brückenglied miteinander verbunden sind.

Aus der GB-PS 1 139 296 ist die Herstellung von 1,4-Bis-(4-chlorbenzoyl)-benzol bekannt. Dabei werden 700 Teile (6,22 mol) Chlorbenzol mit 97 Teilen (0,48 mol) Terephthalsäuredichlorid und 145 Teilen (1,09 mol) gepulvertem wasserfreiem Aluminiumchlorid in 6 Stunden bei 130 °C umgesetzt. Eine Ausbeute wird nicht angegeben. Die analoge Umsetzung von Fluorbenzol ist u.a. in der DE-OS 35 31 837 beschrieben, wobei das Halogenbenzol (hier Fluorbenzol, Aluminiumchlorid und Terephthalsäuredichlorid in praktisch den gleichen Molverhältnissen angewandt werden wie nach der GB-PS 1 139 296. Allerdings ist die Ausbeuteangabe für das 1,4-Bis-(4-fluorbenzoyl)-benzol in der DE-OS 35 31 837 zweifelhaft, da nach der Reinigung mehr Produkt erhalten wird (1250 g) als Rohprodukt (1225 g) vorhanden war. Auch ist der Schmelzpunkt mit 121 °C (statt 218,5 bis 219,5 °C gemäß Hergenrother et al. in Journ. Pol. Sci., Teil A, Polymer Chemistry, 25, 1094 (1987)) falsch angegeben.

Es ist ein Nachteil aller bisher bekannten Verfahren, daß eine große Menge an Aluminiumtrichlorid eingesetzt werden mußte, das bei der Aufarbeitung mit dem Abwasser abgeführt werden mußte und dieses belastete. Gleiches gilt für die übrigen in der DE-OS 35 31 837 genannten Lewis-Säuren Eisenchlorid, Titantetrachlorid und Zinntetrachlorid. Es ist daher wünschenswert, solche Verfahren zu entwickeln, bei denen die Katalysatorsysteme leicht aufgearbeitet und zurückgewonnen werden können.

Gegenstand der vorliegenden Erfindung ist nun ein Verfahren zur Herstellung von halogenhaltigen aromatischen Verbindungen der Formel I (siehe Patentanspruch 1),
worin bedeuten
Hal Halogen,
R H, $C_1$-$C_3$-Alkyl, Phenyl, Halogen oder Trifluormethyl,
Y CO oder $SO_2$ und
Z eine aromatische oder heteroaromatische Gruppierung, wobei Y-Z-Y zusammen auch CO oder $SO_2$ bedeuten können, das dadurch gekennzeichnet ist, daß man einen Halogenaromaten der Formel $RC_6H_4Hal$ (II) mit einem Bissäurehalogenid der Formel Hal-Y-Z-Y-Hal (III) im Molverhältnis von mindestens 2 : 1 in Gegenwart von Fluorwasserstoff und von Bortrifluorid umsetzt, wobei in den Formeln II und III Hal, R, Y und Z die zuvor angegebene Bedeutung haben.

Bevorzugt ist die Herstellung von Verbindungen I, die durch wenigstens eines der Merkmale gekennzeichnet sind, daß Hal Fluor oder Chlor, R Wasserstoff oder Halogen, Y CO und Z Phenylen und insbesondere p-Phenylen ist und daß Halogen in einer anderen als der o-Stellung zu Y steht. Verbindungen mit p-ständigem Halogen sind ganz besonders bevorzugt.

Als Aromaten der Formel II kommen z.B. in Frage Fluorbenzol, o-, m- und p-Difluorbenzol, o-, m- und p-Chlorfluorbenzol, o-, m- und p-Bromfluorbenzol, o-, m- und p-Fluorjodbenzol, o-, m- und p-Fluortoluol, o- und p-Fluorbiphenyl, o- , m-und p-Trifluormethyl-fluorbenzol, Chlorbenzol, o-, m- und p-Dichlorbenzol, o-, m- und p-Bromchlorbenzol, o-, m- und p-Chlorjodbenzol, 4-Chlorbiphenyl, o-, m- und p-Chlortoluol, o-, m- und p-Chlortrifluormethylbenzol, Brombenzol, o-, m- und p-Dibrombenzol, o-, m- und p-Bromjodbenzol, o-, m- und p-Bromtoluol, o-, m- und p-Brombiphenyl, o-, m- und p-Brombenzotrifluorid, Jodbenzol, o- und p-Dijodbenzol, o-, m- und p-Jodtoluol und 3-Jodtrifluormethylbenzol. Bevorzugt sind Brombenzol und ganz besonders Fluorbenzol und Chlorbenzol. Sofern in den Verbindungen $RC_6H_4Hal$ R von Wasserstoff verschieden ist, ist R bevorzugt Halogen.

In den Bissäurehalogeniden III ist Y bevorzugt CO. Z ist z.B. der Rest $C_6H_4$-E-$C_6H_4$ mit E = O, $(CG_2)_m$, CO, S, SO, $SO_2$, worin G Wasserstoff, Methyl, Fluor oder Trifluormethyl und m eine ganze Zahl von 0 bis 4 ist. Wenn m 0 ist, sind die beiden Phenylenreste also durch eine einfache Bindung verbunden.

Als Beispiele für Bissäurehalogenide seien genannt:
Phosgen, Difluorphosgen, Sulfurylchlorid, Sulfurylfluorid, Terephthalsäuredichlorid, Isophthalsäuredichlorid, 1,4- und 2,6-Naphthalindicarbonsäuredichlorid, 1,5-Anthracendicarbonsäuredichlorid, 4,4′-Biphenyldicarbonsäuredichlorid, Bis-(4,4′-chlorcarbonylphenyl)-methan, 2,2-Bis-(4,4′-chlorcarbonylphenyl)-propan, Hexafluor-2,2-bis-(4,4′-chlorcarbonylphenyl)-propan, Di-(4,4′-chlorcarbonylphenyl)keton, Di-(4,4′-chlorcarbonylphenyl)-äther, Di-(4,4-′chlorocarbonylphenyl)-sulfid, Di-(4,4′-chlorcarbonylphenyl)sulfoxid, Di-(4,4′-chlorcarbonylphenyl)-sulfon, Pyridin-2,5-dicarbonsäuredichlorid, 2,5-Thiophendicarbonsäuredichlorid, 1,3- und 1,4-Benzoldisulfonsäuredichlorid, 4,4′-Biphenyldisulfonsäuredichlorid, 2,2-Bis-(4,4′-chlorsulfonylphenyl)-propan, Hexafluor-2,2-bis-(4,4′-chlorsulfonylphenyl)-propan, Di-(4,4′-chlorsulfonylphenyl)äther, Di-(4,4′-chlorsulfonylphenyl)-sulfid, Di-(4,4′-chlorsulfonylphenyl)-sulfoxid, Di-(4,4′-chlorsulfonylphenyl)sulfon und Di-(4,4′-chlorsulfonylphenyl)-keton. Bevorzugt sind Phosgen, Sulfurylchlorid, Terephthalsäuredichlorid,
Di-(4,4′-chlorcarbonylphenyl)-keton,
4,4′-Biphenyldicarbonsäuredichlorid,
Di-(4,4′-chlorcarbonylphenyl)-äther,
Di-(4,4′-chlorcarbonylphenyl)-sulfid,
Di-(4,4′-chlorcarbonylphenyl)-sulfon.

Die als Beispiel genannten Verbindungen der Formel II und der Formel III sind entweder bekannt oder nach analogen Verfahren herstellbar.

Als Endprodukte der Formel I erhält man beispielsweise 4,4′-Difluorbenzophenon, 4,4′-Dichlorbenzophenon, Bis-(4-fluorphenyl)-sulfon, Bis-(4-chlorphenylsulfon), 1,4-Bis″(4-fluorbenzoyl)-benzol, 1,4 Bis-(4-chlorbenzoyl)-benzol, 4,4′-Bis-(4-fluorbenzoyl)diphenyläther, 4,4′-Bis-(4-fluorbenzoyl)-biphenyl, 4,4′-Bis-(4-fluorbenzoyl)-diphenylsulfid und 4,4′-Bis-(4-fluorbenzoyl)-diphenylsulfon.

In dem erfindungsgemäßen Verfahren werden im allgemeinen je Mol Bissäurehalogenid III 2 bis 10, vorzugsweise 2 bis 3 Mol II eingesetzt. Weiterhin werden im allgemeinen je Mol Bissäurehalogenid III 0,1 bzw. 1 bis 100 Mol, vorzugsweise 10 bis 50 Mol und insbesondere 20 bis 30 Mol Fluorwasserstoff eingesetzt. Mengen von weniger als 1 Mol Fluorwasserstoff werden nur dann verwendet, wenn man von einem Bissäurefluorid ausgeht. In diesem Fall ist die Verwendung größerer Mengen Fluorwasserstoff für die Reaktion unkritisch. Es ist dann vorteilhaft, nur verhältnismäßig geringe Mengen Fluorwasserstoff zu verwenden, meistens nicht mehr als 30 und insbesondere 1 bis 5 Mol.

Das erfindungsgemäße Verfahren wird im allgemeinen bei Temperaturen von -80 bis +200°C, vorzugsweise von 0 bis +50°C durchgeführt. Das Bortrifluorid wird im allgemeinen in einer Menge von mindestens 2 Mol je Mol Bissäurehalogenid III verwendet, z.B von 2,5 bis 10 Mol. Man kann bei Atmosphärendruck arbeiten, jedoch ist das Arbeiten bei höherem Druck, insbesondere bei einem Gesamtdruck von 5 bis 70 bar, bevorzugt. Wenn man bei erhöhtem Druck arbeitet, kann man das Verfahren beispielsweise in einem Autoklaven aus Edelstahl, der gegebenenfalls mit einem resistenten Material wie Polytetrafluoräthylen ausgekleidet ist, durchführen, wobei die Ausgangsmaterialien II und III vorgelegt und Fluorwasserstoff zugepumpft wird, z.B. in den oben angegebenen Molmengen, bezogen auf das Bissäurehalogenid III.

Vielfach ist es zweckmäßig, von solchen Bissäurehalogeniden III auszugehen, in denen Hal Fluor ist. Diese Verbindungen brauchen indes nicht als solche eingesetzt zu werden, sondern werden nach einer vorteilhaften Ausführungsform in situ hergestellt, indem solche Verbindungen III, in denen Hal Chlor, Brom oder Jod ist, mit mindestens der äquivalenten Menge Fluorwasserstoff umgesetzt werden. Diese Umhalogenierung kann z.B. bei -20 bis +200°C, vorzugsweise bei 40 bis 80°C, insbesondere bei 50 bis 70°C, durchgeführt werden, z.B. indem man das Gemisch 2 bis 24 Stunden durch Rühren oder Schütteln in inniger Mischung hält. Der dabei entstehende Halogenwasserstoff kann z.B. bei 0 bis 10°C abgedampft werden, gegebenenfalls über eine Kolonne. Danach wird, meistens bei einer Temperatur bis zu 50°C, Bortrifluorid aufgepreßt und das Rohprodukt, das noch Fluorwasserstoff enthält, unter den obengenannten Bedingungen umgesetzt.

Nach einer anderen Ausführungsform können auch die Verbindungen II und III in Fluorwasserstoff vorgelegt werden. Dann wird Bortrifluorid mit einem Druck von 5 bis 70 bar bei den obengenannten Temperaturen aufgepreßt und die Umsetzung zu den Verbindungen I durchgeführt.

Zur Aufarbeitung werden das Bortrifluorid und der Fluorwasserstoff abgedampft. Sie können wieder eingesetzt werden. Das dabei erhaltene Rohprodukt der Formel I kann durch Kristallisation, gegebenenfalls in Gegenwart einer Base wie MgO, $Na_2CO_3$ oder $K_2CO_3$, Waschen oder Destillation oder eine Kombination dieser Maßnahmen gereinigt werden.

Der Vorteil des erfindungsgemäßen Verfahrens besteht darin, daß die Verbindungen II und III in äquimolaren Mengen eingesetzt und der Fluorwasserstoff und das Bortrifluorid ohne Schwierigkeiten zurückgewonnen werden können. Es fällt also bei diesem Verfahren kein Abwasser an, und es entstehen auch keine Emissionen an Schadstoffen, was für ein zeitgemäßes Verfahren von großer Bedeutung ist.

Außerdem wird, im Gegensatz zum bekannten Verfahren, bei dem mit Aluminiumchlorid gearbeitet wird, gemäß der Erfindung ein homogenes Reaktionsgemisch umgesetzt, so daß keine Durchmischungs- bzw. Entmischungsprobleme auftreten.

Die nach dem erfindungsgemäßen Verfahren erhaltenen Bis-(4-halobenzoyl)-Verbindungen, von denen das 1,4-Bis-(4-fluorbenzoyl)-benzol und 1,4-Bis-(4-chlorbenzoyl)-benzol bevorzugt sind, sind wichtige Monomere für chemikalienbeständige Kunststoffe, die auch bei hohen Temperaturen noch beständig sind. Sie können nach Auflösen in geeigneten Lösungsmitteln, gegebenenfalls unter Zusatz fester Basen, vorzugsweise $Na_2CO_3$, $K_2CO_3$ oder MgO, und eventuell nachfolgende Filtration ohne weitere Reinigung direkt für Polykondensationen verwendet werden, z.B. mit Hydrochinon zu Polyätherketonen.

Die Erfindung wird durch die nachfolgenden Beispiele erläutert. Darin bedeutet Schmp. Schmelzpunkt, h Stunde und BFB 1,4-Bis-(4-fluorbenzoyl)-benzol.

## Beispiele

1) In einem 250 ml Autoklaven aus Edelstahl wurden bei 0 °C 20,3 g Terephthalsäuredichlorid, 19,2 g Fluorbenzol und 50 g Fluorwasserstoff vorgelegt und 10 bar Bortrifluorid aufgepreßt. Man schüttelte 16,5 h bei Raumtemperatur. Zur Aufarbeitung wurden die flüchtigen Bestandteile zunächst bei Raumtemperatur und dann bei 60 bis 100 °C abgedampft und der Rückstand aus Chlorbenzol in Gegenwart von Natriumcarbonat umkristallisiert. Man erhielt 19,76 g 1,4-Bis-(4-fluorbenzoyl)-benzol in Form farbloser Kristalle vom Schmp. 221 °C. Bei der Untersuchung mittels Hochdruckflüssigkeitschromatographie (HPLC) ergab sich ein Reinheitsgrad von 99,5 %.

2. Wie in Beispiel 1 wurden 20,3 g Terephthalsäuredichlorid, 21,1 g Fluorbenzol und 50 g Fluorwassertoff bei einem $BF_3$-Druck von 11,5 bar 18 h bei 50 °C umgesetzt. Nach Aufarbeitung erhielt man 20 g BFB (HPLC-Reinheit 99 %).

3. Derselbe Ansatz wie im Beispiel 2 wurde 47 h bei Raumtemperatur umgesetzt. Man erhielt 24,5 g BFB (HPLC-Reinheit 99,5 %).

4. In einem 250 ml Autoklaven aus Edelstahl wurden bei 0 bis 10 °C 20,3 g Terephthalsäuredichlorid, 21,1 g Fluorbenzol und 50 g Fluorwasserstoff vorgelegt und 3 h bei 50 °C geschüttelt. Man kühlte auf 0 °C ab, entspannte den entstandenen HCl-Druck und preßte 10 bar $BF_3$ auf. Man schüttelte 15 h bei Raumtemperatur und erhielt nach Aufarbeitung wie im Beispiel 1 25,6 g BFB (HPLC-Reinheit 99,5 %).

5. Beispiel 4 wurde wiederholt mit der Abweichung, daß bei einem BF3-Druck von 64 bar 16,5 h bei Raumtemperatur geschüttelt wurde. Die Ausbeute an BFB betrug 28,8 g (HPLC-Reinheit 99,5 %).

### Vergleichsversuch 1 (ohne $BF_3$)

In einem 250 ml Autoklaven aus Edelstahl wurden 20,3 g Terephthalsäuredichlorid, 21,1 g Fluorbenzol und 50 g Fluorwasserstoff 24 h bei Raumtemperatur geschüttelt. Nach Aufarbeitung gemäß Beispiel 1 erhielt man kein BFB.

### Vergleichsversuch 2 (ohne HF)

In einem 250 ml Autoklaven aus Edelstahl wurden 20,3 g Terephthalsäuredichlorid und 28,8 g Fluorbenzol vorgelegt, 60 bar Bortrifluorid aufgepreßt und 26 h bei Raumtemperatur geschüttelt. Nach Aufarbeitung gemäß Beispiel 1 erhielt man 2,2 g BFB.

6. In einem 250 mm Schüttelautoklaven aus Edelstahl wurden 34 g (0,2 Mol) Terephthalsäurefluorid, 115 g (1,2 Mol) Fluorbenzol und 4 g (0,2 Mol) wasserfreier Fluorwasserstoff vorgelegt. Man preßte insgesamt 39 g (0,58 Mol) Bortrifluorid auf und schüttelte 24 h bei 20 bis 40 °C. Der Autoklav wurde entspannt und das Reaktionssprodukt - wie im Beispiel 1 beschrieben -aufgearbeitet. Es wurden 54 g (83,8 %) BFB erhalten (HPLC-Reinheit 99 %).

7. In einem 250 ml Schüttelautoklaven aus Edelstahl wurden 17 g (0,1 Mol) Terephthalsäurefluorid, 57,5 g (0,6 Mol) Fluorbenzol und 5 g (0,25 Mol) wasserfreier Fluorwasserstoff vorgelegt. Man arbeitete dann in derselben Weise wie im Beispiel 6 und erhielt 26,5 g (82,4 %) BFB (HPLC-Reinheit 99 %).

## Ansprüche

1. Verfahren zur Herstellung von halogenhaltigen aromatischen Verbindungen der Formel

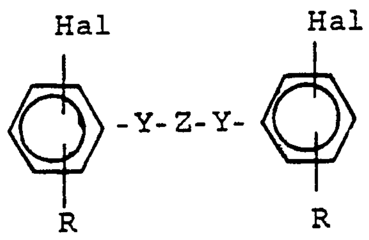

(I),

worin bedeuten
Hal Halogen,
R H, $C_1$-$C_3$-Alkyl, Phenyl, Halogen oder Trifluormethyl, Y CO oder $SO_2$ und
Z eine aromatische oder heteroaromatische Gruppierung, wobei Y-Z-Y zusammen auch CO oder $SO_2$ sein können, dadurch gekennzeichnet, daß man einen Halogenaromaten der Formel $RC_6H_4Hal$ (II) mit einem Bissäurehalogenid der Formel Hal-Y-Z-Y-Hal (III) im Molverhältnis von mindestens 2 : 1 in Gegenwart von Fluorwasserstoff und von Bortrifluorid umsetzt, wobei in den Formeln II und III Hal, R, Y und Z die zuvor angegebene Bedeutung haben.

2. Verfahren gemäß Anspruch 1, dadurch gekennzeichnet, daß 2 bis 10 Mol, vorzugsweise 2 bis 3 Mol, der Verbindung II mit je einem Mol der Verbindung III umgesetzt werden.

3. Verfahren gemäß Anspruch 1 oder 2, dadurch gekennzeichnet, daß Z eine aromatische Gruppierung der Formel $C_6H_4$-E-$C_6H_4$ (IV) ist, worin E O $(CG_2)_m$, CO, S, SO oder $SO_2$, G Wasserstoff, Methyl, Fluor oder Trifluormethyl und m eine ganze Zahl von 0 bis 4 ist.

4. Verfahren gemäß einem oder mehreren der Ansprüche 1 bis 3, gekennzeichnet durch wenigstens eines der Merkmale, daß in den Verbindungen der Formeln I und II Hal Fluor oder Chlor, R Wasserstoff oder Halogen, Y CO und Z Phenylen, insbesondere p-Phenylen, ist und daß Halogen in einer anderen als der o-Stellung zu Y steht.

5. Verfahren gemäß einem oder mehreren der Ansprüche 1 bis 4, dadurch gekennzeichnet, daß als Halogenaromat II Brom-, Chlor- oder insbesondere Fluorbenzol eingesetzt wird.

6. Verfahren gemäß einem oder mehreren der Ansprüche 1 bis 5, dadurch gekennzeichnet, daß Verbindungen der Formel I hergestellt werden, in denen das Halogen in p-Stellung zu Y steht.

7. Verfahren gemäß einem oder mehreren der Ansprüche 1 bis 6, dadurch gekennzeichnet, daß je Mol Bissäurehalogenid III 1 bis 100, vorzugsweise 10 bis 50 und insbesondere 20 bis 30 Mol Fluorwasserstoff eingesetzt werden.

8. Verfahren gemäß einem oder mehreren der Ansprüche 1 bis 7, dadurch gekennzeichnet, daß zunächst ein Bissäurefluorid III eingesetzt wird.

9. Verfahren gemäß Anspruch 8, dadurch gekennzeichnet, daß der Fluorwasserstoff in einer Menge von 0,1 bis 30, insbesondere 1 bis 5 Mol je Mol Bissäurefluorid III verwendet wird.

10. Verfahren gemäß Anspruch 8 oder 9, dadurch gekennzeichnet, daß das Bissäurefluorid III als Rohprodukt, das durch Umsetzung von Bissäurehalogeniden der Formel III, worin Hal Chlor, Brom oder Jod ist, mit mindestens der äquivalenten Menge Fluorwasserstoff bei Temperaturen von -20 °C bis 200 °C, vorzugsweise von 40 bis 80 °C, erhalten wurde.

11. Verfahren gemäß einem oder mehreren der Ansprüche 1 bis 10, dadurch gekennzeichnet, daß die Umsetzung der Verbindungen II und III bei einem Gesamtdruck von 5 bis 70 bar durchgeführt wird.

12. Verfahren gemäß einem oder mehreren der Ansprüche 1 bis 11, dadurch gekennzeichnet, daß man die Umsetzung der Verbindungen II und III bei einer Temperatur von -80 bis 200 °C, vorzugsweise von 0 bis 50 °C durchführt.

13. Verfahren gemäß einem oder mehreren der Ansprüche 1 bis 12, dadurch gekennzeichnet, daß das Bortrifluorid in einer Menge von mindestens 2 Mol, insbesondere von 2,5 bis 10 Mol, je Mol Bissäurehalogenid III verwendet wird.